# FASCICULE DE BREVET EUROPEEN

(11) **EP 2 793 747 B1**
(45) Date de publication et mention de la délivrance du brevet: **02.03.2016**
(21) Numéro de dépôt: 12808415.9
(22) Date de dépôt: 24.12.2012
(51) Int. Cl.: A61F 2/04, A61F 2/90, A61F 5/00

(54) **PROTHÈSE EXPANSIVE DESTINÉE À ÊTRE IMPLANTÉE DANS LE TUBE DIGESTIF D'UN PATIENT**
STENTPROTHESE ZUR IMPLANTATION IM VERDAUUNGSTRAKT EINES PATIENTEN
STENT PROSTHESIS INTENDED TO BE IMPLANTED IN THE DIGESTIVE TRACT OF A PATIENT

(30) Priorité: 23.12.2011 FR 1162442
(43) Date de publication de la demande: 29.10.2014
(73) Titulaire: Assistance Publique - Hôpitaux De Paris, 75475 Paris Cedex 10 (FR)
(72) Inventeur: ATTAR, Alain, F-75017 Paris (FR); BOUHNIK, Yoram, 75009 Paris (FR)
(74) Mandataire: Vidon Brevets & Stratégie
(86) Numéro de dépôt international: PCT/EP2012/076864
(87) Numéro de publication internationale: WO 2013/093112

(56) Documents cités:
- WO-A1-2004/110311
- US-A- 5 876 445
- US-A1- 2011 087 146

## Description

### 1. Domaine de l'invention

Le domaine de l'invention est celui de la conception et de la réalisation de prothèses à usage médical et/ou chirurgical.

Plus précisément, l'invention concerne une prothèse expansible destinée à être implantée dans le tube digestif d'un patient. Le tube digestif est constitué par l'ensemble des organes creux constituant le trajet des aliments depuis la cavité buccale jusqu'au rectum, ce qui comprend l'oesophage, l'estomac, le duodénum, le jéjunum, l'iléon, le caecum, le côlon ascendant, le côlon transverse, le côlon descendant et sigmoïde, le rectum.

Ce type de prothèse est destiné à être implanté pour le traitement de certaines pathologies digestives, et notamment les sténoses digestives courtes, telles que celles survenant dans le cadre d'un cancer, ou de la maladie de Crohn.

### 2. Art antérieur

La maladie de Crohn est une maladie inflammatoire chronique intestinale, probablement d'origine auto-immune. Cette affection se manifeste par des lésions inflammatoires, accompagnées d'ulcérations superficielles ou creusantes, voire de fissures de la membrane, notamment au niveau du colon et de l'iléum. Les données démographiques de la cohorte nationale française CESAME (2004-2005) révèlent que l'intestin grêle est touché chez 70% des patients atteints par la maladie de Crohn. Bien que des traitements médicamenteux existent, le recours à la chirurgie pour ôter les fragments d'intestins ulcérés est quasiment la règle puisque 70% des patients sont opérés. Après une résection, une récidive endoscopique survient jusque dans 90% des cas et peut aboutir à terme à l'apparition d'une sténose.

La sténose est une modification anatomique qui se traduit par le rétrécissement d'une structure tubulaire. Elle aboutit donc à une obstruction totale ou partielle de l'organe creux touché.

Lorsque la sténose affecte le tube digestif, l'obstruction des voies digestives se traduit par un ralentissement du transit intestinal, accompagné de douleurs, l'ensemble pouvant aboutir à une altération de l'état général, une mauvaise absorption des aliments voire à une occlusion intestinale complète.

On peut classer les sténoses en fonction de leur longueur. On distingue alors les sténoses dites « courtes », dont la longueur est inférieure à 4 à 5 cm, et les sténoses longues, dont la longueur est supérieure à 4 à 5 cm. Les sténoses longues nécessitent le recours à la chirurgie pour les exciser. En revanche, les sténoses courtes intestinales peuvent être traitées soit par chirurgie, soit par dilatation endoscopique hydrostatique.

La dilatation endoscopique hydrostatique consiste à faire passer un cathéter dans la lumière de l'intestin du patient sous sédation. Le cathéter contient un ballon qui est progressivement gonflé à l'eau au niveau de la sténose, ce qui permet sa dilatation sous contrôle endoscopique et radiologique. La lumière du tube digestif s'ouvre et la sténose est ainsi traitée. Le ballon est ensuite retiré. Toutefois, la dilatation endoscopique hydrostatique présente un taux de complication entre 1% et 10% (moyenne 2%, Hassan 2007), et un taux de récidive de 30% à 50%. Dans ce dernier cas, le patient subit alors une nouvelle dilatation endoscopique, voire une chirurgie.

Pour améliorer l'efficacité du traitement des sténoses courtes et diminuer le taux de récidives sans avoir recours à la chirurgie, des prothèses tubulaires expansives métalliques ont été proposées. Ces prothèses expansives se présentent sous la forme de tubes à structure en maillage, c'est-à-dire constituées d'un entrelacs de fils, généralement de nature métallique, formant des mailles à la façon d'un grillage. La plupart de ces prothèses se présente sous la forme d'un corps principal tubulaire prolongé à chacune de ses extrémités par une partie tronconique formant une collerette évasée. Ces collerettes, situées de part et d'autre du corps principal, ont un diamètre d'extrémité plus large que celui du corps principal. En revanche, les collerettes sont rigoureusement identiques : elles présentent, entre elles, la même forme et le même diamètre. L'objectif principal de ces collerettes est de permettre un évasement des extrémités de la prothèse afin de permettre de passer plus aisément un endoscope au travers de la prothèse en place si besoin, par exemple pour explorer le tube digestif d'amont.

Les prothèses digestives sont comprimées dans un cathéter pour être amenées au niveau de la sténose sous contrôle endoscopique. Le cathéter les contenant est alors retiré tout en laissant en place la prothèse. Ce retrait permet à la prothèse de se déployer dans le tube digestif, au niveau de la sténose, et de restaurer très rapidement le diamètre du tube et la circulation du bol alimentaire.

Historiquement, les premières prothèses métalliques expansives étaient non recouvertes d'un film polymère quelconque. La principale indication thérapeutique de ces prothèses était le traitement palliatif de sténoses courtes chez des patients souffrant d'un cancer colorectal. En effet, la croissance tumorale finit par obturer la lumière intestinale, affaiblissant ainsi le patient et occasionnant de fortes douleurs. La nature métallique de ces prothèses, sans aucune partie recouverte par un quelconque matériau, leur permet de s'incarcérer dans le tissu digestif. Plus précisément, le tissu, ou la tumeur, se développe autour des mailles de la prothèse. La prothèse se retrouve alors enchâssée dans le tissu digestif et offre ainsi un soulagement durable pour le patient jusqu'à son décès. En ce cas, la prothèse n'est jamais extraite. La pose en intra-prothétique d'une autre prothèse est alors parfois nécessaire du fait de la croissance tumorale.

Plus récemment, d'autres types de prothèses ont été développés afin de soigner des sténoses courtes dans un but de guérison, et non uniquement à titre palliatif.

Ces prothèses sont alors en métal entièrement couvert d'un film polymère, afin d'empêcher leur incarcération et permettre leur retrait. La couverture par un film polymère de la structure en maillage de la prothèse permet de la retirer plus facilement. Toutefois, ce système pose d'autres problèmes.

En effet, ces prothèses sont souvent spontanément et très rapidement éliminées par le patient via les voies naturelles. Le péristaltisme intestinal et la pression exercée par le bol alimentaire provoquent la migration précoce de ces prothèses. Une fois éliminées ou déplacées, ces prothèses ne peuvent plus remplir leur fonction de dilatation de la sténose. Une équipe japonaise a rapporté le cas de patients pour lesquels des prothèses couvertes avaient été posées pour le traitement de sténoses de l'anastomose de résections iléo-coliques consécutives à une maladie de Crohn. Tous les patients ont éliminé leur prothèse par voie rectale (N. Matsuhashi et al., Gastrointestinal Endoscopy, 51 (3), 2000). Une autre étude rapporte que l'utilisation de ce type de prothèse dans l'oesophage a permis de soigner 56% des patients. Toutefois, des cas de migration précoce ont été observés chez 36% des patients (J.C. Bakken et al, Gastrointestinal Endoscopy, 72(4), 2010). Ce phénomène de migration spontanée et précoce a également été observé chez 22% des patients traités pour une sténose consécutive à un cancer colorectal obstructif (G. Fernandez-Esparrach et al. The American Journal of Gastroenterology, 2010). Enfin, une récente étude de patients opérés de l'intestin pour maladie de Crohn et présentant des sténoses anastomostiques a démontré que les prothèses entièrement couvertes migraient de manière précoce chez 70% des patients traités. Parmi ces patients, l'un d'eux a dû subir une dilatation endoscopique de la sténose, afin de récupérer la prothèse qui avait migré précocement en amont de la sténose. Un autre a développé un abcès un mois après la migration (Attar et al, Inflamm Bowel Dis, 2011, sous presse).

Le phénomène de migration spontanée des prothèses n'est pas nécessairement un frein à la dilatation immédiate de la sténose. Il est cependant primordial de maîtriser la durée de la dilatation pour traiter efficacement une sténose. Or, la migration spontanée ne permet pas de contrôler ce paramètre. Il n'est donc pas possible, d'un point de vue médical et éthique, d'implanter une prothèse dont on ne maîtrise pas la durée de mise en place et qui est susceptible d'entraîner des complications (abcès, incarcération etc.).

Par ailleurs, ces prothèses sont coûteuses et leur expulsion par l'organisme du patient limite grandement l'efficacité de leur action. Il est donc nécessaire de concevoir des prothèses qui résolvent notamment le problème de migration précoce des prothèses digestives.

Le document US5876445 décrit une prothèse destinée à être implantée dans le tube digestif d'un patient couverte d'au moins un matériau polymère

### 3. Objectifs de l'invention

L'invention a notamment pour objectif de pallier ces inconvénients de l'art antérieur.

Plus précisément, un objectif de l'invention est de fournir, dans au moins un mode de réalisation, une prothèse digestive qui permette de traiter efficacement les sténoses courtes.

L'invention a encore pour objectif de fournir, dans au moins un mode de réalisation, une prothèse dont l'utilisation permet de supprimer ou, à tout le moins, de limiter grandement le phénomène de migration.

Un autre objectif de l'invention est de proposer, dans au moins un mode de réalisation, une prothèse dont l'utilisation permet de supprimer ou, à tout le moins, de limiter grandement le phénomène d'incarcération.

L'invention a également pour objectif de fournir, dans au moins un mode de réalisation, une prothèse qui soit simple à poser afin de limiter les risques de perforation.

### 4. Exposé de l'invention

Ces objectifs, ainsi que d'autres qui apparaîtront par la suite, sont atteints à l'aide d'une prothèse réalisée en un matériau de base, compressible et expansive dans une direction radiale, destinée à être implantée dans le tube digestif d'un patient.

Selon l'invention, une telle prothèse comprend :
- une collerette aval de forme tronconique présentant un diamètre d'extrémité D3,
- un corps principal tubulaire présentant un diamètre D2,
- une collerette amont de forme tronconique présentant un diamètre d'extrémité D1,
ladite collerette amont étant non recouverte d'un quelconque matériau et présentant un diamètre d'extrémité D1 supérieur au diamètre D2 dudit corps principal et supérieur au diamètre d'extrémité D3 de ladite collerette aval, et ladite collerette aval étant couverte ou constituée, en tout ou partie, par au moins un matériau polymère.

On notera que, dans le cadre de la présente description, les termes « amont » et « aval » sont définis par rapport au sens de migration des aliments dans le tube digestif, c'est-à-dire de la cavité buccale vers le rectum.

Par ailleurs, lorsqu'il est fait référence aux diamètres D1, D2 et D3 de la prothèse, ceux-ci doivent s'entendre des diamètres externes de la prothèse dans son état déployé.

Ainsi, l'invention repose sur une approche tout à fait originale consistant à concevoir une prothèse dont les dimensions des collerettes ne sont pas exactement symétriques de part et d'autre du corps de la prothèse, contrairement aux prothèses actuellement utilisées. L'augmentation du diamètre de la collerette amont, par rapport à l'art antérieur, constitue de fait un frein mécanique. Ce frein permet à la prothèse de résister à la poussée exercée d'une part par le flux d'aliments et de liquides, et d'autre part par la contraction des muscles lisses entourant le tube digestif.

Au sens de l'invention, la collerette amont n'est jamais recouverte par un film polymère. Le matériau constituant la prothèse est donc toujours laissé nu au niveau de la collerette amont. L'absence de matériau de recouvrement sur la collerette amont contribue au meilleur maintien de la prothèse en place, au niveau de la sténose, pour accomplir sa fonction de dilatation. En effet, cette absence de couverture permet au tissu digestif de s'invaginer à travers les mailles de la collerette amont. Ce phénomène contribue à immobiliser la prothèse selon l'invention au niveau de la sténose. De plus, lorsque la prothèse est déployée dans le tube digestif du patient, les parois de la prothèse sont au contact de la muqueuse. Ce contact occasionne des forces de frottements entre la prothèse et la paroi du tube digestif. Ces forces de frottements permettent de freiner le mouvement relatif de la prothèse dans le tube digestif, et donc d'éviter, ou du moins de limiter grandement, la migration de la prothèse au sein du tube digestif.

On comprend dans la description qui va suivre, que l'expression « prothèse partiellement couverte » signifie que la prothèse est recouverte, au moins sur une partie de sa surface, d'un quelconque film polymère.

Préférentiellement, le corps de la prothèse selon l'invention a la forme d'un cylindre de révolution.

Avantageusement, la prothèse selon l'invention présente une structure en maillage.

La structure en maillage permet à la prothèse de passer facilement d'une position rétractée, nécessaire à la pose de la prothèse, à une position déployée fonctionnelle. Ainsi, la prothèse se retrouve plaquée contre la paroi interne du tube digestif. Le déploiement rapide de la prothèse permet de dilater immédiatement la sténose et de rétablir rapidement le transit digestif. Cette structure en maillage permet également la compression de la prothèse afin de l'introduire dans le cathéter et permettre son positionnement sans avoir recours à la chirurgie.

Selon une première variante, ledit matériau de base est un matériau métallique choisi parmi l'acier inoxydable, le titane, le chrome, le nickel, le cobalt ou la combinaison d'au moins deux de ces matériaux, et ladite collerette aval est couverte d'au moins un matériau polymère.

De préférence, le matériau de base est alors un alliage de nickel et de titane tel que le nitinol. Ces matériaux présentent l'avantage d'être bien tolérés par les patients. De plus, ils se révèlent particulièrement résistants au milieu acido-basique environnant, et au contact constant des aliments partiellement digérés. Qui plus est, ces métaux, ou alliages, permettent de produire des matériaux à mémoire de forme. Ils sont donc particulièrement intéressants pour la conception de prothèses expansives et compressibles. Plus particulièrement, grâce à ce type de matériaux combinés à leur structure en maillage, les prothèses peuvent être comprimées dans un cathéter que l'on introduit à travers les cavités naturelles du patient. Ces prothèses sont délivrées à l'emplacement même de la sténose. La libération de la prothèse dans la lumière du tube digestif se traduit par un déploiement immédiat de celle-ci. Ce déploiement est autorisé par l'utilisation d'un matériau à mémoire de forme, ce qui signifie que la prothèse retrouve sa forme initiale déployée lorsque les contraintes exercées par le cathéter disparaissent. De plus, la présence d'une couverture en matériau polymère sur la collerette aval permet de faciliter le retrait de la prothèse. L'absence de résistance lors du retrait facilite non seulement la manipulation par le médecin, mais évite également de blesser ou irriter la paroi digestive du patient. Elle permet donc de limiter le développement d'infections ou de perforations de la paroi digestive, consécutives à la lésion occasionnée par le retrait forcé de la prothèse.

Selon une seconde variante de l'invention, ledit matériau de base constituant la prothèse est un matériau polymère biodégradable, et ladite collerette aval est constituée dudit matériau polymère de base.

Ainsi, la prothèse selon l'invention peut être intégralement constituée d'un matériau polymère biodégradable. L'utilisation d'au moins un matériau biodégradable pour fabriquer la prothèse selon l'invention permet de concevoir une prothèse digestive qui soit à la fois résistante au phénomène de migration et qui puisse rester dans l'organisme du patient jusqu'à sa complète dégradation. Ainsi, le patient n'a pas besoin d'une seconde endoscopie permettant d'extraire la prothèse du tube digestif. Il est en effet préférable de limiter le nombre d'endoscopies chez un même patient. Outre le coût de cet acte, l'endoscopie n'est pas sans risque. La perforation de la paroi digestive par l'endoscope est un risque à prendre en compte. Ce risque est augmenté chez les patients dont la paroi digestive est fragilisée ce qui est notamment le cas lors d'un cancer, d'une maladie de Crohn et de pathologies de la muqueuse en général. Une perforation est une urgence chirurgicale. Certaines équipes de recherche ont commencé à étudier des prothèses oesophagiennes pour le traitement de sténoses bénignes (Y.Saito et al, World J Gastroenterology, 2007, 13(29)). Toutefois, un taux de migration d'environ 77% a été observé parmi les 13 patients traités et ce, quelle que soit l'étiologie de la sténose.

La conception d'une prothèse biodégradable permet en outre de résoudre le problème de leur gestion en tant que déchets médicaux contaminés, ainsi que celui de leur fort impact négatif sur le plan environnemental.

Avantageusement, ledit au moins un matériau biodégradable est choisi parmi le polydioxanone (PDS), l'acide polylactique (PLA), l'acide polylactique lévogyre (PLLA), l'acide polyglycolique (PGA), l'ε-caprolactone.

Ces matériaux polymères sont particulièrement adaptés à la synthèse de prothèses souples. Ils se dégradent dans un délai de quelques semaines, le délai variant en fonction du pH et de la nature des enzymes en présence. Le choix du polymère à mettre en oeuvre dépendra donc, entre autres paramètres, de la destination anatomique de la prothèse et des conditions physico-chimiques qu'elle sera amenée à supporter.

Dans un mode de réalisation préféré, ledit au moins un matériau polymère biodégradable est le polydioxanone (PDS).

Avantageusement, selon cette seconde variante de l'invention selon laquelle le matériau de base de la prothèse est un matériau polymère biodégradable, ladite structure en maillage est réalisée par un tissage ou un tricotage de fils multifilaments ou monofilaments dudit au moins un matériau polymère biodégradable.

Ceci confère à la prothèse une grande résistance mécanique lui permettant de résister aux contraintes qu'elle est amenée à subir après son implantation, et notamment au péristaltisme digestif et au passage du bol alimentaire.

Dans un mode de réalisation avantageux, ledit corps principal tubulaire est couvert, en tout ou partie, d'au moins un matériau polymère. La couverture du corps principal de la prothèse selon l'invention par au moins un matériau polymère permet d'obstruer les éventuelles fistules se produisant au niveau de la sténose. De plus, lorsque la prothèse selon l'invention est réalisée en un matériau métallique, cela permet de faciliter le retrait de la prothèse une fois la sténose correctement dilatée. L'élargissement de la collerette amont suffit alors à retenir la prothèse au niveau de la sténose.

De préférence, ledit matériau polymère est choisi dans le groupe composé du polyuréthane, du polychlorure de vinyle, de l'uréthane, du silicone, du polyamide, du polyester, de la résine fluorique ou la combinaison d'au moins deux de ces matériaux. De préférence, le matériau polymère pouvant recouvrir la prothèse selon l'invention est le silicone.

La présence de cette couche présente deux avantages principaux :
- éviter l'adhésion irréversible de la prothèse à la paroi intestinale, par l'envahissement des mailles de la prothèse par le tissu digestif,
- faciliter ensuite le retrait de la prothèse de l'organisme du patient, sans endommager la paroi digestive au contact de la prothèse.

Ainsi, lorsque la prothèse est constituée d'un matériau métallique, il est particulièrement intéressant de recouvrir la collerette aval et le corps principal de la prothèse d'un matériau polymère tel que le silicone, la résine fluorique, le polyuréthane, le polychlorure de vinyle, l'uréthane, le polyamide, le polyester, ou la combinaison d'au moins deux de ces matériaux. La présence de cette couverture sur le corps principal et la collerette aval permet en effet de faciliter le retrait de la prothèse, une fois la sténose dilatée. Cela permet également de colmater les fistules éventuelles et d'éviter le phénomène d'incarcération de la prothèse dans le tissu. Ainsi, le praticien est certain de ne pas endommager la paroi interne du tube digestif lorsqu'il récupère la prothèse par endoscopie. La couverture du corps de la prothèse, par l'un de ces matériaux, se révèle également intéressante lorsque la prothèse est intégralement réalisée en un matériau polymère biodégradable et que le praticien soupçonne ou a détecté la présence de fistules au niveau de la sténose.

Avantageusement, la paroi de ladite collerette amont selon l'invention forme un angle alpha (α) compris entre 30 degrés et 45 degrés par rapport à l'axe longitudinal de ladite prothèse. De préférence, la paroi de ladite collerette amont selon l'invention forme un angle alpha (α) compris entre 33 degrés et 45 degrés par rapport à l'axe longitudinal de ladite prothèse.

La forte angulation de la collerette amont, par rapport aux prothèses de l'art antérieur, permet de constituer un frein mécanique plus important et plus efficace, sans pour autant endommager la paroi interne du tube digestif. En effet, la paroi des collerettes des prothèses actuelles, et ce depuis une vingtaine d'années, forme un angle compris entre 15 degrés et 22 degrés avec le corps de la prothèse. La valeur de ces angles a toujours été limitée afin de respecter les dimensions anatomiques du tube digestif d'un adulte. Or, les inventeurs ont découvert qu'une prothèse digestive dont la paroi de la collerette amont forme un angle compris entre 30 et 45 degrés permet non seulement d'immobiliser la prothèse au niveau de la sténose, mais ne provoque pas plus de douleurs ou de lésions que les prothèses actuelles. Les inventeurs ont en outre observé que lorsque la prothèse est en alliage métallique avec son corps principal et sa collerette aval recouverts par un matériau polymère, la forte angulation de la collerette amont associée à son diamètre élargi suffit à retenir la prothèse au niveau de la sténose et pallier ainsi le problème de migration des prothèses.

La prothèse selon l'invention présente préférentiellement une longueur totale comprise entre 60 mm et 120 mm.

De façon encore plus préférentielle, la prothèse comprend une longueur totale comprise entre 60 mm et 80 mm.

Ces dimensions sont suffisantes pour traiter les sténoses courtes, c'est-à-dire des sténoses dont la longueur est inférieure ou égale à 5 cm, obturant l'intestin. Il est alors intéressant, lors du placement de la prothèse, de faire en sorte qu'elle dépasse de manière symétrique de part et d'autre de la sténose, si possible d'au moins 2 cm. Le choix de la longueur de la prothèse dépend par conséquent de la longueur de la sténose à traiter.

Avantageusement, ladite collerette amont présente un diamètre d'extrémité D1 compris entre 30 mm et 50 mm.

De préférence, ladite collerette amont présente un diamètre d'extrémité D1 de 40 mm et un angle de 30 degrés.

Ces dimensions permettent de concevoir une collerette amont qui retienne efficacement la prothèse dans le tube digestif, au niveau de la sténose, sans pour autant blesser la paroi digestive ni même induire des sensations désagréables ou douloureuses pour le patient. Ces dimensions sont, par ailleurs, à la fois compatibles avec l'introduction de la prothèse selon l'invention dans un cathéter, et les proportions anatomiques du tube digestif chez un adulte.

Selon un mode de réalisation avantageux, ledit corps tubulaire comprend une longueur comprise entre 20 et 80 mm et un diamètre externe D2 compris entre 15 et 25 mm.

De préférence, la longueur dudit corps tubulaire est comprise entre 24 mm et 60 mm, et son diamètre externe D2 est égal à 20 mm. Ces dimensions sont suffisantes pour dilater une sténose courte et rétablir le transit normal des aliments dans l'intestin.

Avantageusement, ladite collerette aval présente une longueur comprise entre 15 et 25 mm et un diamètre d'extrémité D3 compris entre 25 mm et 32 mm.

De préférence, ladite collerette aval présente une longueur de 18 mm et un diamètre d'extrémité D3 égal à 26 mm.

Une telle collerette participe au maintien de la prothèse dans le tube digestif. La forme évasée de cette collerette, associée à son diamètre légèrement élargi par rapport à celui du corps principal, constitue un second frein mécanique permettant à la prothèse selon l'invention de résister à la pression exercée par le péristaltisme digestif et/ou le bol alimentaire.

Avantageusement, la prothèse selon l'invention comprend au moins un marqueur radio-opaque. L'inclusion de marqueurs radio-opaques radiomarqueurs, ou marqueurs radiologiques ou, sur la surface externe de la prothèse permet son repérage radiologique et la vérification de son positionnement. Ces marqueurs sont libérés au moment de la dégradation de la prothèse et sont éliminés par le patient par les voies naturelles. Ces marqueurs radiologiques peuvent être choisis parmi l'or, le platinum, l'iridium et tout métal biocompatible opaque aux rayons-X....

De préférence, la prothèse comprend deux marqueurs radio-opaques au niveau de chaque collerette et un marqueur radio-opaque au niveau du corps longitudinal. Les marqueurs radiologiques peuvent être fixés sur la prothèse par toute méthode bien connue de l'homme de l'art.

Plus précisément, chacun de ces marqueurs radio-opaques pourra être incorporé au revêtement de la prothèse selon l'invention et/ou être fixé sur le maillage de la prothèse. De préférence, le marqueur est incorporé au revêtement en matériau polymère, lorsque cela est possible.

Dans un mode de réalisation avantageux, la prothèse selon l'invention est recouverte ou inclut dans ledit matériau polymère au moins un principe actif. De préférence, ledit principe actif est choisi parmi un anticorps, une statine, les corticoïdes, une molécule antifibrosante et leur combinaison.

On peut citer à titre d'exemple d'anticorps l'infliximab, adalimumab, le certolizumab, le vedolizumab, l'ustekinumab, le natalizumab. On peut également citer à titre d'exemple de molécule antifibrosante le resveratrol, l'hormone de croissance, les extraits de Scutelleria ou de Boswellia, la mitomycine-C, le CTRP-3 (C1qTNG-related protein 3), anti-TGF-β, l'interleukine 10, le TNRF2, le PEG 15-20, les films biologiques contenant des dérivés de l'acide hyaluronique tels que le chitosan-dextran et le carboxyméthyl-chitosan et le Daikenchuto.

Ainsi, il sera possible de traiter la sténose de manière plus ciblée et d'augmenter le potentiel thérapeutique de la prothèse.

L'invention a encore pour objet une méthode de traitement des sténoses bénignes en général, courtes (mesurant moins de 5-8 cm), les sténoses anastomotiques intestinales et coliques, les sténoses post-opératoires avec ou sans fistules, et toute sténose non néoplasique ou néoplasique avec fistule et ce, quelque soit la partie de tube digestif, pour autant qu'elle soit accessible en endoscopie digestive.

### 5. Liste des figures

D'autres caractéristiques et avantages de l'invention apparaîtront plus clairement à la lecture de la description suivante de modes de réalisation préférentiels, donnés à titre de simple exemple illustratif et non limitatif, et des dessins annexés, parmi lesquels :
- la figure 1 présente une vue de côté d'un premier mode de réalisation d'une prothèse selon l'invention, la prothèse étant totalement déployée ;
- la figure 2 illustre une vue en perspective d'un deuxième mode de réalisation d'une prothèse selon l'invention, la prothèse étant totalement déployée ;
- la figure 3 présente une vue de côté d'une troisième mode de réalisation d'une prothèse partiellement couverte en matériau biodégradable, la prothèse étant totalement déployée ;
- la figure 4 présente une prothèse selon l'invention partiellement déployée, lors de sa mise en place pour le traitement d'une sténose grâce à un cathéter ;
- les figures 5a, 5b, 5c et 5d illustrent différentes étapes de la mise en place d'une prothèse selon l'invention pour le traitement d'une sténose au niveau du colon.

### 6. Description d'un mode de réalisation de l'invention

Le principe général de l'invention consiste en une prothèse digestive compressible et expansive dans une direction radiale. Cette prothèse présente la particularité d'avoir une collerette amont dont le diamètre est élargi, par rapport au corps principal et à la collerette aval de la prothèse. Cette collerette amont élargie forme un frein mécanique permettant de maintenir la prothèse en place.

Selon une variante, lorsque le matériau de base constituant la prothèse est un matériau métallique, l'absence de couverture par un polymère sur la collerette amont, permet au tissu digestif d'être en contact étroit avec les mailles de la prothèse. Ce contact étroit permet à la prothèse de résister à la poussée exercée par les muscles lisses entourant le tube digestif et/ou celle exercée par la progression du bol alimentaire. En revanche, la présence de couverture constituée par un film polymère sur la collerette aval, et éventuellement sur le corps principal de la prothèse, permet non seulement de faciliter le retrait de la prothèse, mais également d'obturer par contact les fistules digestives situées dans la sténose digestive.

Selon une autre variante de l'invention, lorsque le matériau de base constituant la prothèse est un matériau polymère biodégradable, la prothèse selon l'invention est à la fois résistante au phénomène de migration. Elle peut ainsi résister à la migration en dilatant la sténose jusqu'à sa complète dégradation.

Un autre apport de l'invention est de proposer une collerette amont dont la paroi forme un angle α avec l'axe longitudinal du corps de la prothèse largement supérieur à ce qui se pratique actuellement. Cette forte angulation, par rapport à la pratique actuelle, permet de former un frein mécanique plus efficace.

Pour la compréhension des exemples qui vont suivre, les valeurs relatives aux diamètres externes et aux longueurs sont indiquées pour la prothèse dans sa position fonctionnelle, c'est-à-dire lorsqu'elle est entièrement déployée.

### 6.1. Fabrication des prothèses selon l'invention

Les prothèses selon l'invention sont fabriquées selon toute méthode bien connue de l'homme de l'art. Le procédé de fabrication de telles prothèses ne fait pas l'objet de la présente demande. En bref, les fils de matériau polymère biodégradable ou de matériau métallique, par exemple à mémoire de forme, sont associés ensemble pour former un maillage. Le maillage est ensuite découpé au laser afin d'obtenir une coupe franche et nette des fils. Une portion de maillage ainsi obtenue est ensuite conformée pour obtenir la forme de la prothèse.

### 6.2. Prothèse digestive destinée à être implantée chez un patient.

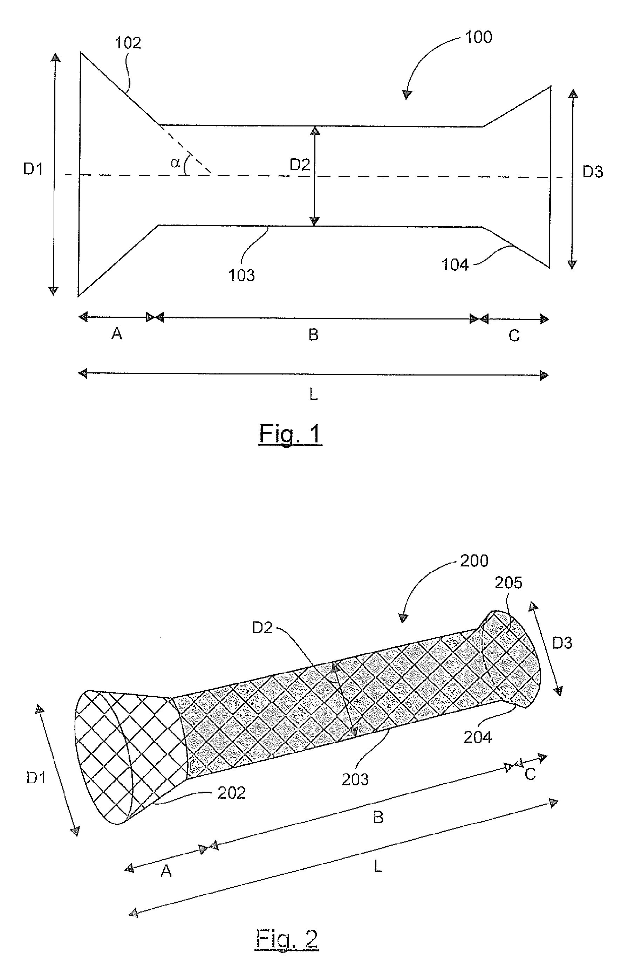

On présente, en relation avec la figure 1, le schéma d'une vue de profil de la prothèse selon l'invention, la prothèse étant représentée totalement déployée. La prothèse 100 présente une forme globalement tubulaire, avec une structure en maillage (non représentée sur la figure 1). Elle est composée de trois parties principales : un corps tubulaire 103 et deux collerettes 102, 104 disposées de part et d'autre du corps tubulaire 103. Chacune des collerettes 102, 104 présente une forme tronconique, évasée vers l'extérieur, vu de profil, lorsqu'elle est déployée.

La collerette amont 102 forme un angle alpha (α) avec l'axe longitudinal du corps principal tubulaire 103 de la prothèse, l'angle α étant compris entre 30 degrés et 45 degrés. Afin de faciliter la compréhension de l'invention, l'axe longitudinal est représenté en pointillé sur la figure 1. Cette collerette amont 102 présente un diamètre d'extrémité D1 compris entre 30 et 50 mm. Préférentiellement, la collerette amont 102 a un diamètre d'extrémité d'au moins 40 mm et forme un angle α compris entre 30 degrés et 45 degrés avec le corps de la prothèse. De préférence, l'angle α est compris entre 33 degrés et 45 degrés. Il est important que cette collerette 102 soit du plus grand diamètre possible, tout en respectant les contraintes d'insertion dans un cathéter ainsi que les dimensions anatomiques du tube digestif. En effet, il n'est pas souhaitable que le diamètre trop important de la collerette 102 provoque une souffrance au patient, voire qu'elle abîme la paroi du tube digestif. Il est également important que l'angle α que forme la paroi de la collerette amont 102 avec le corps de la prothèse soit compris entre 30 degrés et 45 degrés. La longueur A de la collerette amont 102 sera calculée en fonction du diamètre d'extrémité et de l'angulation qu'on souhaite obtenir pour cette collerette amont.

Le corps tubulaire 103, qui a préférentiellement la forme d'un cylindre de révolution, présente une longueur B et un diamètre externe D2. Cette longueur B est comprise entre 20 mm et 80 mm. De préférence, la longueur du corps tubulaire est comprise entre 24 mm et 60 mm. Le diamètre externe D2 du corps 103 est compris entre 15 et 25 mm.

La collerette aval 104 présente une longueur C comprise entre 15 mm et 25 mm, et un diamètre d'extrémité D3 compris entre 25 mm et 32 mm.

Avantageusement, les extrémités libres des collerettes amont 102 et aval 104 sont arrondies, pour éviter de blesser ou irriter la paroi du tube digestif.

L'épaisseur de la prothèse étant égale à celle des fils du matériau la constituant, les diamètres internes des différentes parties composant la prothèse sont légèrement inférieurs à celui de leur diamètre externe. La longueur totale de la prothèse L est la somme des longueurs A, B et C.

Selon une première variante, la prothèse est réalisée dans un maillage constitué d'un entrelacs de fils métalliques préférentiellement en alliage à mémoire de forme. Ce maillage n'est recouvert d'aucun revêtement, le métal étant laissé à nu. Selon une seconde variante, la prothèse est réalisée en un matériau polymère biodégradable. Selon chacune de ces variantes, la prothèse peut alors être partiellement recouverte par un film en matériau polymère, à la surface de sa collerette aval, mais également à la surface de son corps principal tubulaire.

La structure en maillage, ou grillagée, permet à la prothèse d'entrer en contact étroit avec le tissu digestif, occasionnant des frottements. Cette structure en maillage permet en outre d'obtenir une prothèse compressible et expansible dans une direction radiale, par rapport à l'axe longitudinal du corps de la prothèse. Les dimensions de la prothèse seront choisies en fonction de l'application pour laquelle la prothèse est mise en oeuvre et en particulier en fonction des dimensions anatomiques de l'organe à traiter.

### 6.3. Prothèse digestive métallique partiellement couverte destinée à être implantée chez un patient.

On présente en relation avec la figure 2 un mode de réalisation préféré de la prothèse selon l'invention. La prothèse 200 présente une structure en maillage. Ce maillage est réalisé en nitinol, qui est un alliage à partir de nickel et de titane. Ce matériau est particulièrement souple et possède la particularité de recouvrer sa forme après avoir été compressé. Grâce à la structure en maillage et aux caractéristiques mécaniques du nitinol, la prothèse peut être comprimée dans un cathéter. Le retrait du cathéter entourant la prothèse permet à cette dernière de se déployer et de recouvrir sa forme initiale très rapidement.

La prothèse 200 est constituée :
- d'une collerette amont 202, ayant une forme tronconique,
- d'un corps 203 ayant la forme d'un cylindre de révolution, et
- d'une collerette aval 204 ayant également une forme tronconique.

La collerette amont 202 présente un diamètre D1 de 40 mm de diamètre et forme un angle α avec l'axe longitudinal de la prothèse égal à 33 degrés. Pour des raisons de clarté, l'angle α n'a pas été représenté sur cette figure. Le corps de la prothèse 203 présente une longueur B de 44 mm et un diamètre D2 égal à 20 mm. La collerette aval 204 présente une longueur C de 18 mm et un diamètre externe D3 de 26 mm. La collerette 202 est laissée non couverte, tandis que le corps 203 et la collerette aval 204 sont recouverts d'un film polymère de silicone 205, recouvrant la surface externe de tous le corps de la prothèse. La présence du film 205 de silicone à la surface du corps 203 et de la collerette 204 permet non seulement de faciliter le retrait de la prothèse, mais également d'éviter son incarcération dans la muqueuse digestive. L'absence de couverture par le film polymère sur la collerette amont 202 permet à la prothèse d'adhérer de manière réversible à la paroi intestinale. En effet, la prothèse étant déployée dans l'intestin, les parois de la prothèse sont au contact de la muqueuse intestinale. L'absence de couverture à la surface du maillage constituant la prothèse l'empêche de glisser contre la paroi. Elle permet en outre au tissu digestif de coloniser les mailles de la prothèse, aidant à l'accrochage de celle-ci en amont de la sténose. Ceci contribue donc à éviter, ou du moins à limiter, la migration de la prothèse au sein du tube digestif. De cette manière, le procédé anti-migration constitué par l'angulation de la collerette, le diamètre élargi et l'absence de couverture de la collerette amont permettent d'avoir une durée de dilatation contrôlée suffisamment longue pour éviter la récidive sténotique.

### 6.4 Prothèse digestive en polydioxanone partiellement couverte destinée à être implantée chez un patient.

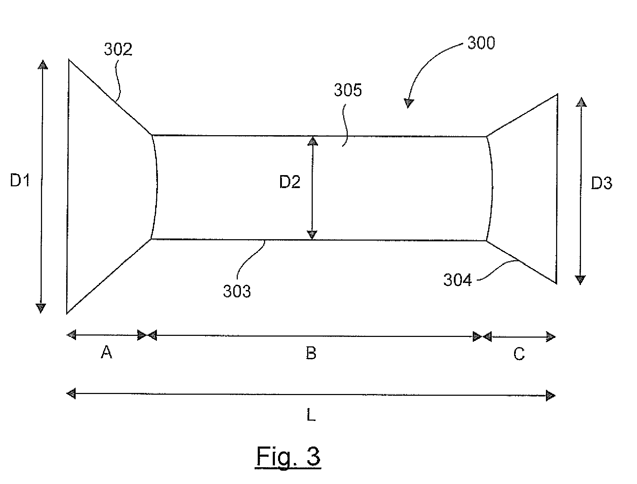

Dans une variante particulièrement avantageuse de l'invention, on décrit en relation avec la figure 3 une prothèse en matériau en polymère biodégradable présentant une structure en maillage (non représentée). Ce maillage est réalisé en polydioxanone. Grâce à la structure en maillage, la prothèse peut être comprimée dans un cathéter. Le retrait du cathéter entourant la prothèse permet à cette dernière de se déployer et de recouvrir sa forme initiale très rapidement.

La prothèse 300 est constituée :
- d'une collerette amont 302, ayant une forme tronconique,
- d'un corps 303 ayant la forme d'un cylindre de révolution, et
- d'une collerette aval 304 ayant également une forme tronconique.

La collerette amont 302 présente un diamètre d'extrémité D1 de 40 mm de diamètre et forme un angle α avec l'axe longitudinal de la prothèse égal à 33 degrés. Pour des raisons de clarté, l'angle α n'a pas été représenté sur cette figure. Le corps de la prothèse 303 présente une longueur B de 44 mm et un diamètre externe D2 égal à 20 mm. La collerette aval 304 présente une longueur C de 18 mm et un diamètre d'extrémité D3 de 26 mm. La collerette amont 302 et la collerette aval 304 sont laissées non couvertes, tandis que le corps 303 est recouvert d'un film polymère de silicone 305, recouvrant la surface externe de la prothèse. L'absence de couverture à la surface du maillage de la collerette amont 302, associé à son diamètre élargi et à la forte angulation par rapport à l'axe longitudinal de la prothèse, l'empêche de glisser contre la paroi. Ce frottement participe au maintien en place de la prothèse dans le tube digestif du patient et contribue donc à éviter, ou du moins à limiter grandement, la migration de la prothèse biodégradable au sein du tube digestif. De plus, l'absence de couverture au niveau de la collerette amont permet au tissu digestif de s'introduire dans les orifices créés par le maillage constituant la prothèse. Ce phénomène physiologique et normal permet de retenir la prothèse au niveau de la sténose. Ainsi, la durée de dilation de la sténose est parfaitement contrôlée et les complications liées à la migration de la prothèse (extraction difficile, risque de perforation etc...) sont évitées.

De plus, la couverture par un film polymère du corps principal tubulaire de la prothèse permet d'obstruer les éventuelles fistules situées dans la sténose.

### 6.5. Insertion d'une prothèse digestive partiellement couverte destinée à être implantée chez un patient souffrant d'une sténose courte au niveau du côlon, par la méthode du largage distal.

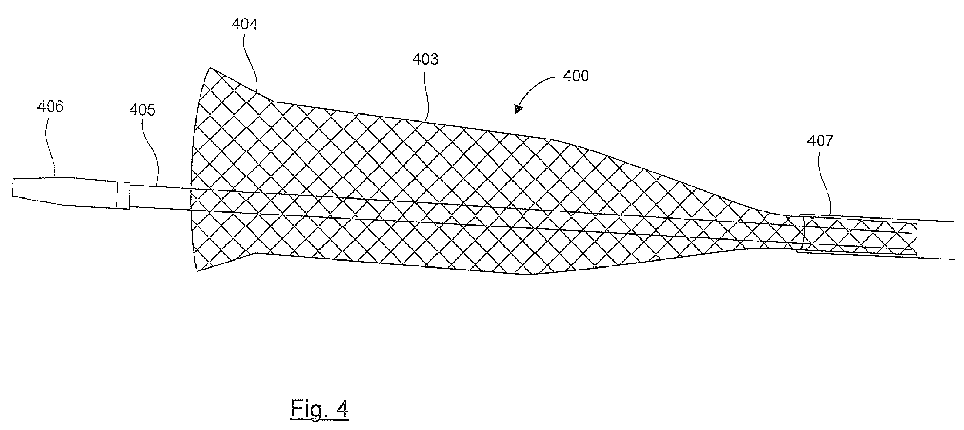

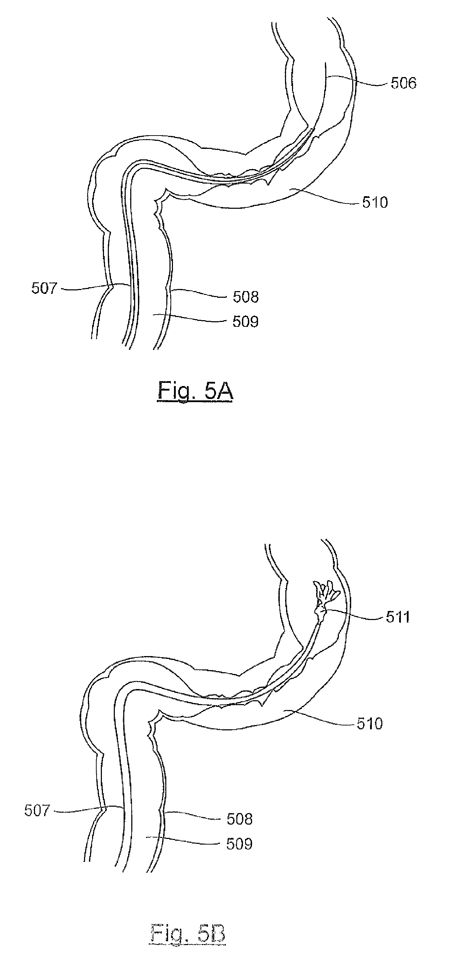

On décrit en relation avec les figures 4 et 5A-5D la pose d'une prothèse, selon l'exemple 6.3 ou 6. 4, chez un patient souffrant d'une sténose courte (longueur < 5 cm), au niveau de l'anastomose consécutive à une résection iléo-colique pour maladie de Crohn. Cette prothèse est non couverte au niveau de la collerette amont. Le corps de la prothèse et/ou la collerette aval pourra/pourront être couverts par un matériau polymère, préférentiellement en silicone.

Comme représenté à la figure 5A, une endoscopie classique est tout d'abord réalisée, selon toute méthode bien connue de l'homme de l'art, afin de visualiser l'emplacement de la sténose 510 au niveau de l'intestin 508 du patient. Une fois la sténose 510 repérée, un fil guide 506 souple est introduit dans la lumière intestinale 509, à travers la lumière de l'endoscope 507. Le fil guide 506 est acheminé de manière à franchir la sténose 510, c'est-à-dire que le fil guide traverse toute la zone rétrécie par la sténose jusqu'à arriver à une zone de l'intestin exempte de sténose. Sur ce guide, un cathéter est glissé en amont de la sténose et le guide est retiré pour opacification.

Le produit de contraste iodé 511, par exemple du Telebrix®, est injecté dans la lumière d'amont de l'intestin via le cathéter, l'endoscope étant toujours en aval de la sténose, comme schématisé à la figure 5B. Ce produit de contraste sert à visualiser l'amont pour bien repérer l'emplacement de la sténose, et le positionnement correct de l'endoscope et du cathéter contenant la prothèse par radioscopie.

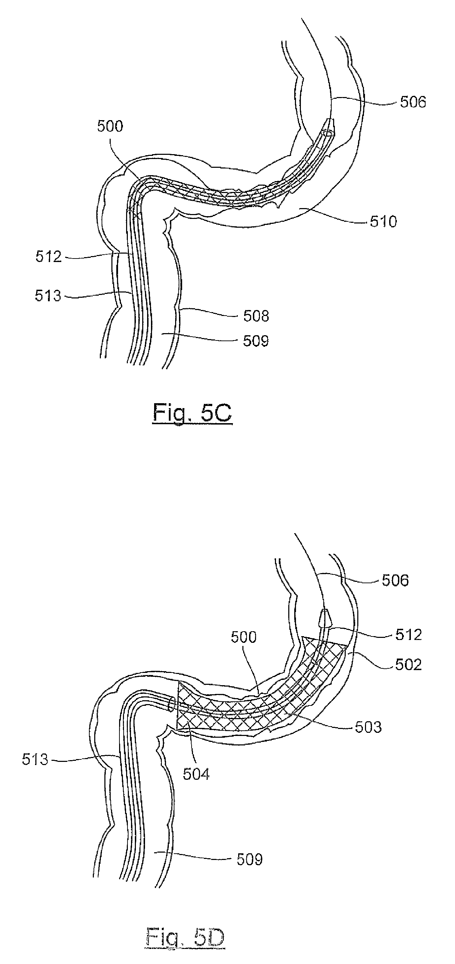

Le cathéter 513 contenant la prothèse 200 est introduit dans le canal opérateur de l'endoscope, toujours en place en aval de la sténose, et passé sous contrôle endoscopique et radiologique au travers de la lumière de la sténose intestinale via le guide 509, comme représenté à la figure 5C. Ce cathéter contient un conduit interne 512, à l'intérieur duquel peut circuler le fil guide 506. La prothèse 500, identique à la prothèse 200 ou la prothèse 300, est comprimée entre les parois du cathéter 513 et les parois du conduit interne 512. Cette prothèse 500 comprend une collerette amont 502, un corps principal 503 et une collerette aval couverte 504. L'ensemble cathéter - prothèse - conduit interne est poussé dans la lumière intestinale 509 du patient, jusqu'à atteindre la sténose 510. L'ensemble est poussé de manière à positionner la prothèse 500, en la faisant dépasser de part et d'autre de la sténose 510. Comme schématisé à la figure 5D, le cathéter 513 est ensuite retiré pour permettre à la prothèse 500 de se déployer dans l'intestin du patient. Ce déploiement immédiat est permis par la structure en maillage et l'utilisation d'un matériau à mémoire de forme. Le fil 506 et le conduit intérieur 512 entourant le fil sont ensuite retirés pour ne laisser que la prothèse 500 au niveau de la sténose 510.

La figure 4 présente une vue agrandie et schématisé de la prothèse selon l'invention lors du retrait du cathéter, ce retrait permettant de libérer la prothèse au niveau de la sténose. Telle que représentée sur cette figure, la prothèse 400, identique à celle décrite à la figure 2 ou 3, est partiellement contenue dans un cathéter 407. Ce schéma permet de mieux observer le phénomène de déploiement de la structure en maillage, dès que la prothèse est dégagée de l'emprise du cathéter. Un fil de guidage 405 circule dans la prothèse 400, de manière sensiblement parallèle à l'axe longitudinal de celle-ci. Le fil de guidage 405 est souple et sert à introduire puis orienter un endoscope ou le cathéter 407 contenant la prothèse. L'extrémité distale 406 de ce fil de guidage 405 est lubrifiée pour faciliter son insertion et son cheminement dans le tube digestif du patient. On entend par « extrémité distale » l'extrémité qui est insérée dans l'organisme du patient. Cette caractéristique du guide et sa souplesse permettent d'éviter la formation de lésion ou de perforation d'aval en ayant permis le cathétérisme de l'intestin d'amont sur une longueur suffisante par l'opérateur.

Les prothèses sont ensuite retirées au bout de 7 jours, la sténose étant stabilisée dans sa dilatation par la prothèse. L'extraction se fait de la façon suivante : une coloscopie est à nouveau réalisée comme indiqué précédemment. Le coloscope est monté jusqu'au niveau de la sténose qui laisse voir la collerette aval de la prothèse, toujours en place dans sa sténose maintenant dilatée. Les deux lassos attenants à 3h et 9h sur les bords de la collerette aval sont saisis par une pince endoscopique et tractés vers l'endoscope permettant de fermer cette collerette comme une bourse et la déformant comme une ogive. Ceci permet d'attirer cette collerette ainsi déformée via la pince vers le bas, la guidant au contact de l'endoscope jusqu'à descente à l'extérieur du patient. Il peut parfois être utile d'utiliser aussi un lasso inséré au milieu de l'intérieur du corps de la prothèse pour mieux tracter l'ensemble, toujours à l'aide d'une pince ; le lasso central permettant une invagination de la prothèse en son centre, évitant par là même des lésions muqueuses à la traction de l'ensemble.

### 6.6. Essai clinique chez deux patients adultes souffrant d'une sténose courte au niveau du côlon.

Deux prothèses selon l'invention ont été posées chez deux patients, tous deux atteints par la maladie de Crohn, âgés de 36 ans et 56 ans. Chacun de ces patients a subi des résections chirurgicales iléo-coliques suite au développement de leur maladie. Des sténoses courtes, inférieures à 5 cm, se sont développées au niveau de l'anastomose de ces résections. L'un des patients présentait une sténose récidivante, 6 mois après dilatation endoscopique.

Comme décrit au point 6.3, la collerette amont de chacune des prothèses est laissée non couverte et présente un diamètre extérieur de 40 mm et forme un angle α de 33 degrés avec l'axe longitudinal de la prothèse. La collerette aval de chacune des prothèses est recouverte par un film de silicone et présente un diamètre externe de 26 mm et une longueur de 18 mm. Le corps de la prothèse mesure 44 mm de longueur pour un diamètre externe de 20 mm, et est également recouvert d'un film de silicone. Les prothèses ont été posées comme décrit au point 6.5, sans aucune complication. Les prothèses ont ensuite été retirées au bout de 7 jours. Au terme de ce délai, aucune migration précoce n'a été observée, c'est-à-dire que chacun des patients a conservé sa prothèse et qu'elle ne s'est pas déplacée dans l'intestin. Dans les deux cas, le syndrome sub-occlusif a cédé immédiatement après la pose de la prothèse. Par ailleurs, alors qu'une résistance est généralement observée lors du retrait d'une prothèse ayant, par exemple, ses deux collerettes non couvertes, le retrait de la prothèse selon l'invention s'est parfaitement déroulé.

La survenue de récidive a été surveillée tous les mois par la clinique et l'imagerie médicale. Aucune récidive n'a été constatée dans les 3 mois qui ont suivi le retrait de la prothèse. Aucune sténose résiduelle ou dilatation n'a été observée chez ces patients.

### 7. Conclusions

La prothèse selon l'invention permet donc de traiter les sténoses courtes, quelle que soit leur étiologie. L'association du diamètre élargi et l'absence de recouvrement de la collerette amont par un quelconque matériau permet d'éviter, ou à tout le moins de réduire considérablement, le problème de migration précoce, qui est un risque majeur avec les prothèses actuellement sur le marché. La présence d'une couverture par un matériau polymère de la collerette aval permet de faciliter le retrait de la prothèse et d'éviter de blesser la paroi digestive des patients lors de cette manipulation. La présence éventuelle de couverture par un polymère au niveau du corps de la prothèse permet en outre de faciliter le retrait de la prothèse, et même de limiter le phénomène d'incarcération. De plus, la forte angulation de la collerette amont, par rapport aux prothèses de l'art antérieur, constitue un frein mécanique extrêmement efficace, sans pour autant endommager la paroi interne du tube digestif ou occasionner des douleurs pour le patient.

Ainsi, les inconvénients liés à l'utilisation des prothèses expansives pour le traitement des sténoses courtes sont maintenant capables d'être éliminés grâce à la prothèse selon l'invention, ou à tout le moins d'être fortement atténués.

L'absence de recouvrement par un polymère sur au moins la collerette amont pouvait faire penser qu'il existait un risque d'incarcération à ce niveau. Or, les essais *in vivo* ont démontré que ce phénomène n'avait jamais été observé.

Particulièrement, une prothèse selon l'invention réalisée en un matériau de base métallique et présentant une collerette amont élargie et non recouverte par un quelconque matériau, ainsi qu'un corps principal et une collerette aval recouverts par un matériau polymère, permet de résoudre à la fois les problèmes de migration et d'incarcération observés. La présence du film polymère permet en outre d'obstruer les fistules éventuelles qui se produisent parfois, notamment suite à des sutures.

Par ailleurs une prothèse selon l'invention réalisée en un matériau de base métallique et présentant une collerette amont élargie et non recouverte par un quelconque matériau, un corps principal non recouvert par un quelconque matériau et une collerette aval recouverte par un matériau polymère trouverait son intérêt pour le traitement palliatif de sténoses chez des patients souffrant d'un cancer colorectal. Une telle prothèse est également plus souple, du fait de la faible portion de sa surface couverte par un matériau polymère. Elle permet donc de résoudre le problème de perforation de la paroi digestive.

Enfin, selon une seconde variante de l'invention une prothèse réalisée en un matériau de base constitué par un matériau polymère biodégradable permet à la fois de résoudre le problème de migration, le problème de perforation et éventuellement, le problème d'incarcération. En outre, elle ne produit aucun déchet et son impact environnemental est plus faible que celui des prothèses métalliques.

Ainsi, tout ou partie des inconvénients liés à l'utilisation des prothèses expansives pour le traitement des sténoses courtes sont maintenant capables d'être éliminés, ou à tout le moins d'être fortement atténués.

On notera qu'il pourra être envisagé d'autres modes de réalisation de l'invention. Notamment, il est possible d'ajouter des radiomarqueurs opaques ou des marqueurs fluorescents à la surface de la prothèse pour permettre sa visualisation en radioscopie ou fluoroscopie. On peut également prévoir de pré-équiper la prothèse selon l'invention avec un fil guide, circulant de manière sensiblement parallèle à l'axe longitudinal de la prothèse, pour faciliter son insertion et son positionnement à travers la sténose. Enfin, on peut prévoir l'association de valvules anti-reflux pour des applications oesophagiennes.

Il pourra également être envisagé de recouvrir la prothèse selon l'invention, ou d'inclure dans la couverture de matériau polymère, d'au moins un principe actif, de préférence un principe actif thérapeutique. Ainsi le potentiel thérapeutique de la prothèse selon l'invention s'en trouvera accru.

De plus, bien que les essais cliniques exposés ci-dessus ne concernent que le traitement de sténoses coliques, ces prothèses sont tout à fait adaptées au traitement de sténoses oesophagiennes, pyloriques, trachéo-bronchiques... Il suffit, pour ce faire, d'adapter les proportions relatives des différentes parties de la prothèse afin qu'elle s'insère dans la structure anatomique que l'on désire traiter.

Il a été décrit, dans la présente demande un largage distal de la prothèse et par voie endoscopique basse dans le tube digestif du patient, à savoir que la prothèse à été introduite dans le tube digestif du patient par l'anus (i.e. endoscopie dite par voie basse). Distal et proximal s'entendent par rapport à la vision endoscopique : la prothèse se déploie par son extrémité (collerette) la plus éloignée de l'endoscope en largage distal et par son extrémité (collerette) la plus proche de l'endoscope en largage proximal. Il est entendu que la prothèse selon l'invention peut également être placée chez un patient par endoscopie dite par voie haute, en passant par la bouche du patient. Par cette voie, la méthode de largage (i.e. déploiement de la prothèse quand on mobilise son cathéter porteur) pourra être distale, ce qui est qui est le cas le plus fréquemment utilisé en endoscopie digestive, ou bien proximale. Si l'on utilise la voie haute avec largage distal, la technique n'est pas différente de ce qui a été décrit précédemment en voie basse : on tire sur le cathéter et la prothèse se déploie par son extrémité distale par rapport à l'endoscope. En revanche, en voie haute avec largage proximal, le cathéter sera poussé de l'amont vers l'aval pour libérer la prothèse. Une fois la prothèse totalement déployée, il suffit de retirer le cathéter vide avec son guide. Quelque soit le type de largage en voie haute, la prothèse devra être manufacturée de façon à ce que la collerette anti-migration soit toujours la plus proche de l'endoscope dans son cathéter porteur pour que ce soit elle qui soit en amont de la sténose à traiter, eu égard au sens du flux et de la motricité digestive.

## Revendications

1. Prothèse (100, 200, 300, 400, 500), compressible et expansive dans une direction radiale, destinée à être implantée dans le tube digestif d'un patient, ladite prothèse (100, 200, 300, 400, 500) étant réalisée en un matériau de base et comprenant :
- une collerette aval (104, 204, 304, 404, 504) de forme tronconique présentant un diamètre d'extrémité D3,
- un corps principal tubulaire (103, 203, 303, 403, 503) présentant un diamètre D2,
- une collerette amont (102, 202, 302, 402, 502) de forme tronconique présentant un diamètre d'extrémité D1,
ladite collerette amont (102, 202, 302, 402, 502) étant non recouverte d'un quelconque autre matériau et présentant un diamètre d'extrémité D1 supérieur au diamètre D2 dudit corps principal (103, 203, 303, 403, 503) et supérieur au diamètre d'extrémité D3 de ladite collerette aval (104, 204, 304, 404, 504), et ladite collerette aval étant couverte ou constituée, en tout ou partie, d'au moins un matériau polymère.

2. Prothèse (100, 200, 300, 400, 500) selon la revendication 1 **caractérisée en ce qu'**elle présente une structure en maillage.

3. Prothèse (100, 200, 400, 500) selon la revendication 2 dans laquelle ledit matériau de base est un matériau métallique choisi parmi l'acier inoxydable, le titane, le chrome, le cobalt ou la combinaison d'au moins deux de ces matériaux, et en ce que ladite collerette aval (104, 204, 404, 504) est couverte d'au moins un matériau polymère

4. Prothèse (100, 300, 400, 500) selon la revendication 1 ou 2 **caractérisée en ce que** ledit matériau de base est un matériau polymère biodégradable et **en ce que** ladite collerette aval (104, 304, 404, 504) est constituée dudit matériau polymère de base.

5. Prothèse (100, 300, 400, 500) selon la revendication 4 dans laquelle ledit au moins un matériau polymère biodégradable est choisi parmi le polydioxanone (PDS), l'acide polylactique (PLA), l'acide polylactique lévogyre (PLLA), l'acide polyglycolique (PGA), l'ε-caprolactone.

6. Prothèse (100, 300, 400, 500) selon la revendication 4 ou 5 dans laquelle ledit au moins un matériau polymère biodégradable est le polydioxanone.

7. Prothèse (100, 300, 400, 500) selon l'une des revendications 4 à 6 ladite structure en maillage est réalisée par un tissage ou un tricotage de fils multifilaments ou monofilament dudit au moins un matériau polymère biodégradable.

8. Prothèse (100, 200, 300, 400, 500) selon l'une des revendications précédentes **caractérisée en ce que** ledit corps principal tubulaire (103, 203, 303, 403, 503) est couvert, en tout ou partie, d'au moins un matériau polymère.

9. Prothèse (100, 200, 300, 400, 500) selon la revendication 3 ou 8 **caractérisée en ce que** ledit matériau polymère est choisi dans le groupe composé du polyuréthane, du polychlorure de vinyle, de l'uréthane, du silicone, du polyamide, du polyester, de la résine fluorique, du polytetrafluoroethylène ou la combinaison d'au moins deux de ces matériaux.

10. Prothèse (100, 200, 300, 400, 500) selon l'une quelconque des revendications précédentes **caractérisée en ce que** la paroi de ladite collerette amont (102, 202, 302, 402, 502) forme un angle alpha (α) compris entre 30 degrés et 45 degrés par rapport à l'axe longitudinal de ladite prothèse.

11. Prothèse (100, 200, 300, 400, 500) selon l'une des revendications précédentes **caractérisée en ce que** ladite prothèse présente une longueur totale comprise entre 60 mm et 120 mm.

12. Prothèse (100, 200, 300, 400, 500) selon l'une des revendications précédentes **caractérisée en ce que** ladite collerette amont (102, 202, 302, 402, 502) présente un diamètre d'extrémité D1 compris entre 30 mm et 50 mm.

13. Prothèse (100, 200, 300, 400, 500) selon l'une des revendications précédentes **caractérisée en ce que** ledit corps principal tubulaire (103, 203, 303, 403, 503) comprend une longueur comprise entre 20 mm et 80 mm et un diamètre externe D2 compris entre 15 mm et 25 mm.

14. Prothèse (100, 200, 300, 400, 500) selon l'une des revendications précédentes **caractérisée en ce que** ladite collerette aval (104, 204, 304, 404, 504) présente une longueur comprise entre 15 mm et 25 mm et un diamètre d'extrémité D3 compris entre 25 mm et 32 mm.

15. Prothèse selon l'une des revendications précédentes **caractérisée en ce qu'**elle comprend au moins un marqueur radio-opaque.

16. Prothèse selon l'une des revendications précédentes recouverte ou incluant dans ledit matériau polymère au moins un principe actif.

## Patentansprüche

1. Prothese (100, 200, 300, 400, 500), die in eine Radialrichtung komprimierbar und expandierbar ist, die dazu bestimmt ist, im Verdauungstrakt eines Patienten implantiert zu werden, wobei die Prothese (100, 200, 300, 400, 500) aus einem Basismaterial hergestellt ist und umfasst:
- einen vorderen Kragen (104, 204, 304, 404, 504) von kegelstumpfartiger Form, der einen Enddurchmesser D3 aufweist,
- einen röhrenförmigen Hauptkörper (103, 203, 303, 403, 503), der einen Durchmesser D2 aufweist,
- einen hinteren Kragen (102, 202, 302, 402, 502) von kegelstumpfartiger Form, der einen Enddurchmesser D1 aufweist,
wobei der hintere Kragen (102, 202, 302, 402, 502) nicht mit irgendeinem Material bedeckt ist und einen Enddurchmesser D1 größer als der Durchmesser D2 des Hauptkörpers (103, 203, 303, 403, 503) und größer als der Enddurchmesser D3 des vorderen Kragens (104, 204, 304, 404, 504) aufweist, wobei der vordere Kragen zur Gänze oder zum Teil aus mindestens einem Polymermaterial gebildet oder von diesem bedeckt ist.

2. Prothese (100, 200, 300, 400, 500) nach Anspruch 1, **dadurch gekennzeichnet, dass** sie eine Maschenstruktur aufweist.

3. Prothese (100, 200, 400, 500) nach Anspruch 2, bei der das Basismaterial ein metallisches Material ist, das unter rostfreiem Stahl, Titan, Chrom, Kobalt oder der Kombination von mindestens zwei dieser Materialien ausgewählt ist, und dass der vordere Kragen (104, 204, 404, 504) mit mindestens einem Polymermaterial bedeckt ist.

4. Prothese (100, 300, 400, 500) nach Anspruch 1 oder 2, **dadurch gekennzeichnet, dass** das Basismaterial ein biologisch abbaubares Polymermaterial ist, und dass der vordere Kragen (104, 304, 404, 504) aus dem Basispolymermaterial gebildet ist.

5. Prothese (100, 300, 400, 500) nach Anspruch 4, bei der das mindestens eine biologisch abbaubare Polymermaterial unter Polydioxanon (PDS), Polymilchsäure (PLA), linksdrehender Polymilchsäure (PLLA), Polyglycolsäure (PGA), ε-Caprolacton ausgewählt ist.

6. Prothese (100, 300, 400, 500) nach Anspruch 4 oder 5, bei der das mindestens eine biologisch abbaubare Polymermaterial Polydioxanon ist.

7. Prothese (100, 300, 400, 500) nach einem der Ansprüche 4 bis 6, wobei die Maschenstruktur durch Weben oder Stricken von Multifilamenten oder Monofilamenten des mindestens einen biologisch abbaubaren Polymermaterials hergestellt ist.

8. Prothese (100, 200, 300, 400, 500) nach einem der vorhergehenden Ansprüche, **dadurch gekennzeichnet, dass** der röhrenförmige Hauptkörper (103, 203, 303, 403, 503) zur Gänze oder zum Teil mit mindestens einem Polymermaterial bedeckt ist.

9. Prothese (100, 200, 300, 400, 500) nach Anspruch 3 oder 8, **dadurch gekennzeichnet, dass** das Polymermaterial in der Gruppe ausgewählt ist, bestehend aus Polyurethan, Polyvinylchlorid, Urethan, Silikon, Polyamid, Polyester, Fluorharz, Polytetrafluorethylen oder der Kombination aus mindestens zwei dieser Materialien.

10. Prothese (100, 200, 300, 400, 500) nach einem der vorhergehenden Ansprüche, **dadurch gekennzeichnet, dass** die Wand des vorderen Kragens (102, 202, 302, 402, 502) einen Winkel Alpha (α) zwischen 30 Grad und 45 Grad in Bezug zur Längsachse der Prothese bildet.

11. Prothese (100, 200, 300, 400, 500) nach einem der vorhergehenden Ansprüche, **dadurch gekennzeichnet, dass** die Prothese eine Gesamtlänge zwischen 60 mm und 120 mm aufweist.

12. Prothese (100, 200, 300, 400, 500) nach einem der vorhergehenden Ansprüche, **dadurch gekennzeichnet, dass** der hintere Kragen (102, 202, 302, 402, 502) einen Enddurchmesser D1 zwischen 30 mm und 50 mm aufweist.

13. Prothese (100, 200, 300, 400, 500) nach einem der vorhergehenden Ansprüche, **dadurch gekennzeichnet, dass** der röhrenförmige Hauptkörper (103, 203, 303, 403, 503) eine Länge zwischen 20 mm und 80 mm und einen Außendurchmesser D2 zwischen 15 mm und 25 mm aufweist.

14. Prothese (100, 200, 300, 400, 500) nach einem der vorhergehenden Ansprüche, **dadurch gekennzeichnet, dass** der vordere Kragen (104, 204, 304, 404, 504) eine Länge zwischen 15 mm und 25 mm und einen Enddurchmesser D3 zwischen 25 mm und 32 mm aufweist.

15. Prothese nach einem der vorhergehenden Ansprüche, **dadurch gekennzeichnet, dass** sie mindestens einen strahlungsundurchlässigen Marker umfasst.

16. Prothese nach einem der vorhergehenden Ansprüche, die mit mindestens einem Aktivprinzip bedeckt ist oder ein solches in dem Polymermaterial einschließt.

## Claims

1. Prosthesis (100, 200, 300, 400, 500), that is compressible and expandable in a radial direction, intended to be implanted in the digestive tract of a patient, said prosthesis (100, 200, 300, 400, 500) being made from a base material and comprising:
- a downstream frustoconical collar (104, 204, 304, 404, 504) having an end diameter D3,
- a main tubular body (103, 203, 303, 403, 503) having a diameter D2,
- a upstream frustoconical collar (102, 202, 302, 402, 502) having an end diameter D1,
said upstream collar (102, 202, 302, 402, 502) not being covered by any other material and having an end diameter D1 greater than the diameter D2 of said main body (103, 203, 303, 403, 503) and greater than the end diameter D3 of said downstream collar (104, 204, 304, 404, 504), and said downstream collar being covered or constituted, fully or partially, by at least one polymer material.

2. Prosthesis (100, 200, 300, 400, 500) according to claim 1 **characterised in that** it has a mesh structure.

3. Prosthesis (100, 200, 400, 500) according to claim 2 wherein said base material is a metal material chosen from stainless steel, titanium, chromium, cobalt or the combination of a least two of these materials, and in that said downstream collar (104, 204, 404, 504) is covered by at least one polymer material.

4. Prosthesis (100, 300, 400, 500) according to claim 1 or 2 **characterised in that** said base material is a biodegradable polymer material and **in that** said downstream collar (104, 304, 404, 504) is constituted of said polymer-base material.

5. Prosthesis (100, 300, 400, 500) according to claim 4 wherein said at least one biodegradable polymer material is chosen from among polydioxanone (PDS), polylactic acid (PLA), poly-L-lactic acid (PLLA), polyglycolic acid (PGA), ε-caprolactone.

6. Prosthesis (100, 300, 400, 500) according to claim 4 or 5 wherein said at least one biodegradable polymer material is polydioxanone.

7. Prosthesis (100, 300, 400, 500) according to one of claims 4 to 6 said mesh structure is carried out by a weaving or knitting of multifilament or monofilament threads of said at least one biodegradable polymer material.

8. Prosthesis (100, 200, 300, 400, 500) according to one of the preceding claims **characterised in that** said main tubular body (103, 203, 303, 403, 503) is covered, fully or partially, by at least one polymer material.

9. Prosthesis (100, 200, 300, 400, 500) according to claim 3 or 8 **characterised in that** said polymer material is chosen from the group comprised of polyurethane, polyvinyl chloride, urethane, silicone, polyamide, polyester, fluorine resin, polytetrafluoroethylene or the combination of at least two of these materials.

10. Prosthesis (100, 200, 300, 400, 500) according to any of the preceding claims **characterised in that** the wall of said upstream collar (102, 202, 302, 402, 502) forms an angle alpha (α) between 30 degrees and 45 degrees in relation to the longitudinal axis of said prosthesis.

11. Prosthesis (100, 200, 300, 400, 500) according to one of the preceding claims **characterised in that** said prosthesis has a total length between 60 mm and 120 mm.

12. Prosthesis (100, 200, 300, 400, 500) according to one of the preceding claims **characterised in that** said upstream collar (102, 202, 302, 402, 502) has an end diameter D1 between 30 mm and 50 mm.

13. Prosthesis (100, 200, 300, 400, 500) according to one of the preceding claims **characterised in that** said main tubular body (103, 203, 303, 403, 503) comprises a length between 20 mm and 80 mm and an outside diameter D2 between 15 mm and 25 mm.

14. Prosthesis (100, 200, 300, 400, 500) according to one of the preceding claims **characterised in that** said downstream collar (104, 204, 304, 404, 504) has a length between 15 mm and 25 mm and an end diameter D3 between 25 mm and 32 mm.

15. Prosthesis according to one of the preceding claims **characterised in that** it comprises at least one radiopaque marker.

16. Prosthesis according to one of the preceding claims covering or including in said polymer material at least one active ingredient.
